# EUROPEAN PATENT SPECIFICATION

(11) **EP 3 065 799 B1**
(45) Date of publication and mention of the grant of the patent: **08.01.2020**
(21) Application number: 14799648.2
(22) Date of filing: 05.11.2014
(51) Int. Cl.: A61M 5/148, A61M 5/142, A61M 5/162, A61K 9/00, A61M 31/00, A61M 5/145

(54) **FLUID DELIVERY DEVICES AND METHODS**
FLÜSSIGKEITSABGABEVORRICHTUNGEN UND VERFAHREN
DISPOSITIFS ET PROCEDES DE DISTRIBUTION D'UN FLUIDE

(30) Priority: 05.11.2013 US 201314071929
(43) Date of publication of application: 14.09.2016
(73) Proprietor: Inguran, LLC, Navasota, TX 77868 (US)
(72) Inventor: ABHISHEK, Ramkumar, Mountain View, California 94040-2012 (US); LINN, Felicia, San Jose, California 95112 (US)
(74) Representative: Jacob, Reuben Ellis
(86) International application number: PCT/US2014/064030
(87) International publication number: WO 2015/069700

(56) References cited:
- EP-A1- 2 308 546
- WO-A1-99/07346
- WO-A2-2011/133724
- WO-A2-2013/040247
- US-A1- 2009 281 528
- US-A1- 2014 088 345
- US-A1- 2014 088 346

## Description

### FIELD

The present disclosure is generally in the field of fluid delivery devices and methods, and more particularly to devices and methods for the controlled delivery of one or more fluids, including but not limited to implantable drug delivery devices.

### BACKGROUND

Controlled fluid delivery is desirable in a variety of applications, including drug delivery. However, it would be desirable to provide improved devices and methods to independently store and effectively deliver one or more fluids from a single device to a target area. For example, it would be desirable to provide a delivery system capable of separately storing two or more fluids onboard a device, yet including shared means for controlled fluid release from the device in a compact, space-efficient device. Examples of said kind of devices or similar devices can be found in US 2014/088346, US 2014/088345, WO 2013/040247, EP 2 308 546, US 2009/281528, WO 2011/133724 and WO 99/07346.

### SUMMARY

In one aspect, a fluid delivery device is provided, including: (i) a main housing having an interior region and having an outer surface which includes a porous membrane, (ii) a first fluid reservoir disposed in the interior region of the main housing, the first fluid reservoir being formed at least in part by a first wall structure and containing a first fluid, (iii) a first puncture mechanism operable to puncture the first wall structure and form a fluidic path between the first fluid reservoir and one or more first channels, which are in fluid communication with the porous membrane, and (iv) a first positive displacement mechanism operable, following puncture of the first wall structure, to drive the first fluid out of the first fluid reservoir, through the one or more first channels, and into the porous membrane. The porous membrane may be configured to distribute the first fluid to an area adjacent to and outside of the outer surface of the main housing.

In another aspect, an implantable drug delivery device for controlled release of two or more drugs to an intralumenal tissue surface is provided, the device including: (i) a main housing configured for intralumenal deployment in a patient, the main housing having an outer surface which includes a porous membrane, (ii) a first fluid reservoir within the main housing and containing a first fluid which includes a first drug, (iii) a second fluid reservoir within the main housing and containing a second fluid which includes a second drug, (iv) a first puncture mechanism operable to puncture the first fluid reservoir and form a fluidic path between the first fluid reservoir and one or more first channels, which are in fluid communication with the porous membrane, (v) a second puncture mechanism operable to puncture the second fluid reservoir and form a fluidic path between the second fluid reservoir and one or more second channels, which are in fluid communication with the porous membrane, and (vi) at least one positive displacement mechanism operable to (a) drive the first fluid out of the first fluid reservoir, through the one or more first channels, and into the porous membrane following puncture of the first fluid reservoir, and (b) drive the second fluid out of the second fluid reservoir, through the one or more second channels, and into the porous membrane following puncture of the second fluid reservoir. The porous membrane may be operable to distribute the first and second drugs to an intralumenal tissue surface adjacent to the outer surface of the main housing.

In yet another aspect, a method of controlled delivery of a fluid to a target area is provided, the method including: (i) positioning a fluid delivery device adjacent to the target area, wherein the fluid delivery device includes: (a) a first fluid reservoir defined by a first fluid reservoir housing, the first fluid reservoir containing a first fluid, (b) a first wall structure forming at least a portion of the first fluid reservoir housing, and (c) a porous membrane forming an outer surface of the fluid delivery device, (ii) puncturing the first wall structure with a first puncture mechanism to provide a fluidic path between the first fluid reservoir and one or more first channels in fluid communication with the porous membrane, and (iii) driving the first fluid out of the first fluid reservoir and into the one or more first channels, such that the first fluid is delivered to the target area from the device via the porous membrane.

### BRIEF DESCRIPTION OF THE DRAWINGS

FIG. 1A is a cross-sectional view of one embodiment of a fluid delivery device having a single fluid reservoir, in a tissue lumen, prior to puncturing the wall structure.
FIG. 1B is a cross-sectional view of the fluid delivery device of FIG. 1A, after puncturing the wall structure and prior to driving the fluid out of the fluid reservoir.
FIG. 1C is a cross-sectional view of the fluid delivery device of FIG. 1A, while driving the fluid out of the fluid reservoir and into the channels to deliver the fluid to the tissue surface via the porous membrane.
FIG. 2A is a cross-sectional view of one embodiment of a fluid delivery device having two fluid reservoirs, prior to puncturing the wall structure of either reservoir.
FIG. 2B is a cross-sectional view of the fluid delivery device of FIG. 2A, after puncturing the wall structure of the first fluid reservoir and while driving the fluid out of that reservoir.
FIG. 2C is a cross-sectional view of the fluid delivery device of FIG. 2A, after puncturing the wall structure of the second fluid reservoir and while driving the fluid out of that reservoir.
FIG. 3A is an exploded perspective view of one embodiment of a fluid delivery device having two fluid reservoirs and two independent sets of channels in fluid communication with a porous membrane.
FIG. 3B is a perspective view of the fluid delivery device of FIG. 3A.
FIG. 4A is a perspective view of one embodiment of an implantable drug delivery device having a porous membrane sidewall.
FIG. 4B is an exploded perspective view of the drug delivery device of FIG. 4A.
FIG. 5A is a cross-sectional view of one embodiment of a fluid delivery device having a single fluid reservoir, in a tissue lumen, prior to puncturing the wall structure.
FIG. 5B is a cross-sectional view of the fluid delivery device of FIG. 5A, after puncturing the wall structure and prior to driving the fluid out of the fluid reservoir.
FIG. 5C is a cross-sectional view of the fluid delivery device of FIG. 5A, while driving the fluid out of the fluid reservoir and into the channels to deliver the fluid to the tissue surface via the porous membrane.

### DETAILED DESCRIPTION

The fluid delivery devices and methods described herein provide for the storage and controlled delivery of one or more fluids from a single device. The devices are advantageously configured to separately store and isolate the fluid(s), thereby minimizing the risk of contamination, and to precisely dispense the fluid(s) according to a desired release timing profile. For example, these devices and methods may be used to deliver drugs to a patient in vivo. The devices and methods disclosed herein can significantly increase the accuracy and efficiency of delivering multiple fluids, in a space-efficient manner, e.g., with minimal duplication of the fluid delivering components, and without unwanted mixing of the fluids to be released.

In certain embodiments, the fluid delivery devices include a porous membrane to distribute the fluid(s) to the area adjacent the device. However, the devices disclosed herein may also be constructed without the porous membrane. For example, a device may have a common gate or valve other than a porous membrane, which is configured to distribute the fluid(s) to the area adjacent the device.

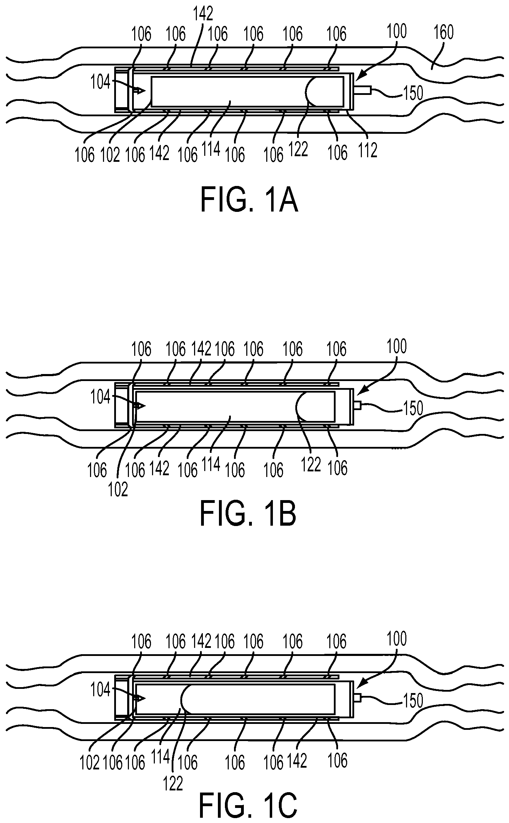

In one aspect, a fluid delivery device is provided. As shown in FIG. 1A, in one embodiment the device **100** includes a main housing **112** having an interior region and having an outer surface which includes a porous membrane **142.** A first fluid reservoir **114** is disposed in the interior region of the main housing **112,** and is formed at least in part by a first wall structure **102.** The first fluid reservoir **114** contains a first fluid. The device **100** also includes a first puncture mechanism **104** operable to puncture the first wall structure **102** and form a fluidic path between the first fluid reservoir **114** and one or more first channels **106,** which are in fluid communication with the porous membrane **142.** The device **100** also includes a first positive displacement mechanism **122** operable, following puncture of the first wall structure **102,** to drive the first fluid out of the first fluid reservoir **114,** through the one or more first channels **106,** and into the porous membrane **142.** The porous membrane **142** is configured to distribute the first fluid to an area adjacent to and outside of the outer surface of the main housing **112.**

In another aspect, a method of controlled fluid delivery to a target area is provided. In certain embodiments, as shown in FIGS. 1A-1C the method includes: (i) positioning a fluid delivery device **100** adjacent to the target area, (ii) puncturing the first wall structure **102** with a first puncture mechanism **104** to provide a fluidic path between the first fluid reservoir **114** and one or more first channels **106** in fluid communication with the porous membrane **142,** and (iii) driving the first fluid out of the first fluid reservoir **114** and into the one or more first channels **106,** such that the first fluid is delivered to the target area from the device **100** via the porous membrane **142.**

Various embodiments and features of the fluid delivery devices and methods are described in greater detail hereinafter.

### MAIN HOUSING

The device includes a main housing having an interior region and an outer surface. In certain embodiments, the outer surface of the main housing includes a porous membrane. Generally, these devices are configured to deliver one or more fluids to an area adjacent to and outside of the outer surface of the main housing.

The main housing of the device may be shaped and dimensioned for a specific fluid delivery application. For example, as shown in FIG. 1A, the device **100** may be an implantable medical device shaped and dimensioned for intralumenal deployment into a human or animal subject. The term "intralumenal," as used herein, refers to placement within a body cavity, channel, tube, or the like, having a mucosal wall. The term includes, but is not limited to, sites in the reproductive tract, such as intravaginal, cervical, or intrauterine, and the gastrointestinal tract, such as intrarectal.

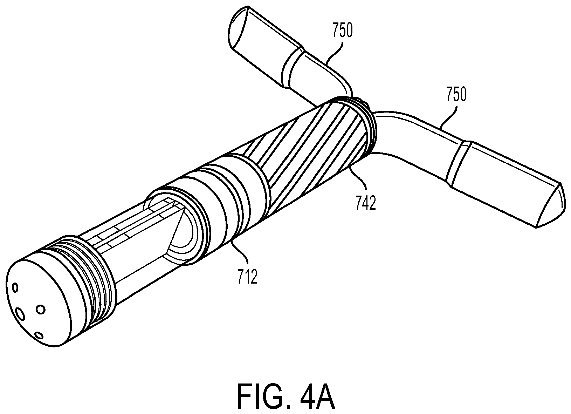

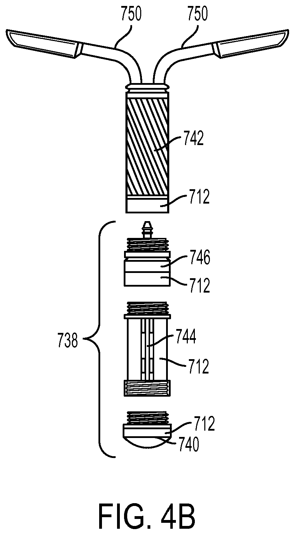

The housing configuration may be based upon the particular lumenal site and human or animal anatomical considerations, for deployment with minimal discomfort to the patient. In certain embodiments, the device may be placed within the lumen by insertion into the lumen via an exterior body orifice. Accordingly, in certain embodiments, the housing is shaped and dimensioned to allow insertion and placement, i.e., deployment, of the device within the intended lumen via the exterior body orifice. For example, the housing may be shaped and dimensioned for vaginal, cervical, uterine, or rectal insertion and placement. As shown in FIGS. 4A and 4B, the housing **712** may include an elongated, substantially cylindrical outer surface and have wing-like portions, or arms, **750** extending therefrom. For example, this configuration may be appropriate for vaginal device deployment in livestock, such as cattle, sheep, etc.

The materials of construction, size, shape, surface features, and other characteristics of the housing may be configured such that the device can be deployed into the lumen, retained securely in the lumen during operation of the device for an extended period of time, and retrieved from the lumen following operation of the device or when otherwise desired to be removed. For example, the device may be removed between the delivery of individual drug formulations, following the delivery of several drug formulations, or following the completion of a course of treatment of multiple drug formulations. The device may be deployed until the drug formulation payload is depleted.

In certain embodiments, the housing is formed of a biocompatible material. Moreover, the housing material may be resistant to degradation in the mucosal environment of the lumen. Examples of suitable housing materials include stainless steel, titanium, and biocompatible polymers, such as polypropylene, polyethylene, or other common polymers, such as nylon having a biocompatible outer layer, e.g., silicone. The housing material may include a coating to enhance biocompatibility and/or operation of the device.

### RESERVOIRS AND CONTENTS

At least one reservoir is disposed within the main housing of the device and contains a fluid for delivery from the device. The reservoir(s) may be shaped and dimensioned to fit within the main housing and may be formed of materials that are suitable for storing the fluid to be contained therein. Examples of suitable reservoir materials include stainless steel, titanium, and biocompatible polymers, such as polypropylene, polyethylene, or other common polymers, such as nylon having a biocompatible outer layer, e.g., silicone. In certain embodiments, the reservoir may be formed at least in part by the inner surface of the main housing.

As shown in FIG. 1A, the reservoir **114** is formed at least in part by a first wall structure **102.** The first wall structure may include a puncturable material. Examples of suitable wall structure materials include elastomeric polymers or other self-sealing materials, such as silicone, PTFE, and synthetic and natural rubber, and combinations thereof.

In certain embodiments, the device includes two or more reservoirs, with each reservoir containing a fluid to be delivered and being formed at least in part by a wall structure. In particular embodiments, the fluids are drugs that are selected to work in concert, and beneficially are delivered in parallel or series, for example in a separated or overlapping schedule. In certain embodiments, multiple reservoirs may contain a similar fluid to be delivered in multiple administrations. For example, the device may include multiple reservoirs containing the same drug to be administered in discrete doses to a patient.

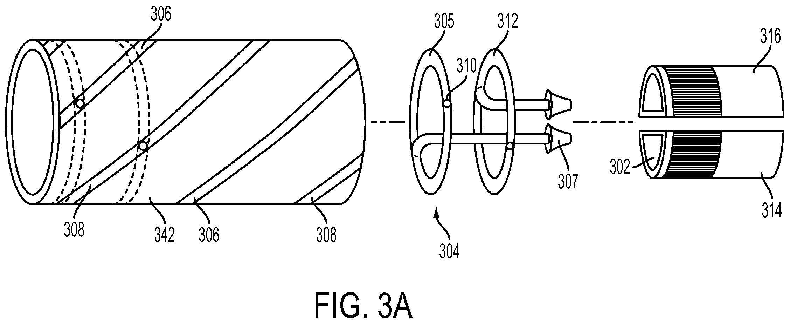

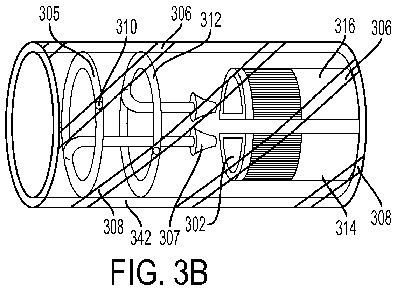

The reservoirs may be disposed within the housing such that they are parallel to the housing and/or each other. The reservoirs may each be defined by an inner surface of an elongated annular tube. The reservoirs may also have a combined shape similar to that of the housing and be configured such that the reservoirs occupy a majority of the volume of the housing. In certain embodiments, the reservoirs are elongated and have a circular cross-sectional shape. In certain embodiments, the reservoirs together have a circular cross-sectional shape, with each reservoir having a cross-sectional shape that forms a portion of the circular shape. In one embodiment, as shown in FIGS. 3A and 3B, each of two reservoirs **314, 316** has a semicircular cross-sectional shape such that the reservoirs together form a circular cross-sectional shape. Other cross-sectional shapes are also envisioned. In certain multi-reservoir embodiments, at least two of the reservoirs are configured to have different volumes.

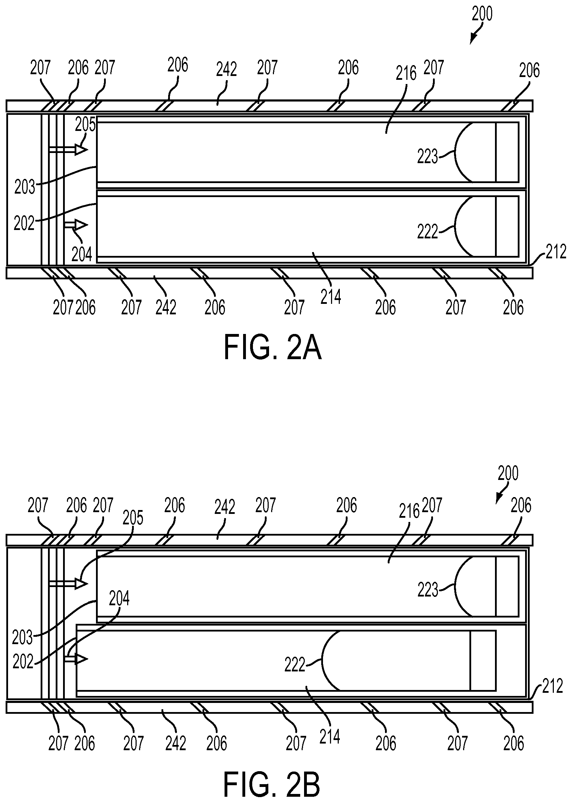

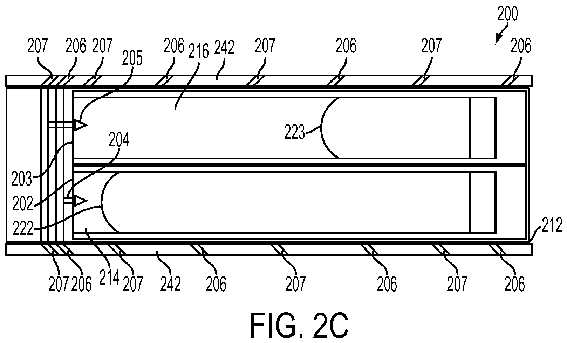

As shown in FIG. 2A, the device **200** includes a first reservoir **214** containing a first fluid and a second reservoir **216** containing a second fluid. Both reservoirs **214, 216** are disposed in the interior region of the main housing **212.** The first reservoir **214** is formed in part by a first wall structure **202** and the second reservoir **216** is formed in part by a second wall structure **203.** In certain embodiments, the first and second fluid reservoirs each include an elongated annular tube with a closed end wall.

In certain embodiments, each reservoir has an actuation end and an opposed release end. The actuation ends may be operably connected to an actuation system. The release ends may include the wall structure that is configured to be punctured by the puncture mechanism. The actuation end of each reservoir includes a positive displacement mechanism that is operable, following puncture of the reservoir's wall structure, to drive the fluid out of the reservoir. As shown in FIGS. 1A-1C, the device **100** includes a first positive displacement mechanism **122** operable, following puncture of the first wall structure **102,** to drive the first fluid out of the first fluid reservoir **114,** through the one or more first channels **106,** and into the porous membrane **142.** In embodiments having multiple reservoirs, each reservoir may be associated with a separate positive displacement mechanism, as shown in FIGS. 2A-2C, or two or more reservoirs may share a positive displacement mechanism.

In certain embodiments, the positive displacement mechanism includes an expandable membrane. For example, the expandable membrane may include an inflatable balloon structure that expands or inflates to drive the fluid out of the reservoir. The balloon structure may be an elastomer, such as latex, nitrile or urethane based, or it may be a collapsed balloon, such as a thin metallized polymer sheet, e.g., polyester or polyethylene.

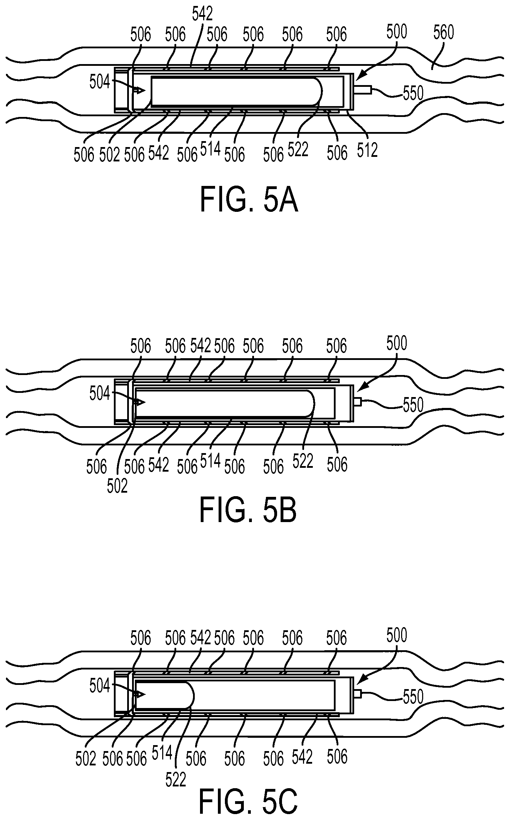

In other embodiments, as shown in FIGS. 5A-5C, the fluid is contained within a balloon **522** disposed in the reservoir **514,** and the positive displacement mechanism includes a gas generating mechanism configured to generate a gas to deflate the balloon and displace the first fluid. As shown in FIG. 5A, in one embodiment the device **500** includes a main housing **512** having an interior region and having an outer surface which includes a porous membrane **542.** A first fluid reservoir **514** is disposed in the interior region of the main housing **512,** and is formed at least in part by a first wall structure **502.** The first fluid reservoir **514** at least partially contains balloon **522,** which contains a first fluid. The device **500** also includes a first puncture mechanism **504** operable to puncture the first wall structure **502** and form a fluidic path between the first fluid reservoir **514** and/or the balloon **522** and one or more first channels **506,** which are in fluid communication with the porous membrane **542.** The device **500** also includes positive displacement mechanism operable, following puncture of the first wall structure **502** (shown in FIG. 5B), to generate a gas in communication with the balloon **522,** to deflate the balloon **522** and drive the first fluid out of the first fluid reservoir **514,** through the one or more first channels **506,** and into the porous membrane **542,** as shown in FIG. 5C. The porous membrane **542** is configured to distribute the first fluid to an area adjacent to and outside of the outer surface of the main housing **512.**

Alternatively, the positive displacement mechanism may include a plug or plunger structure configured to advance through the reservoir and drive the fluid out of the reservoir. For example, the plugs may sealingly engage with, and slide with respect to, the inner walls of the reservoirs. The plugs may function as pistons.

For example, the devices described herein may include one or more of the reservoir and/or main housing features described in U.S. Patent Applications No. 13/629,124 and 13/629,159.

### RELEASE STRUCTURE

In embodiments, the device is configured to deliver the fluid(s) to a target area adjacent the outer surface of the main housing. To initiate the release of the fluid from a reservoir, the wall structure of the reservoir is punctured by the puncture mechanism and the positive displacement mechanism is actuated to drive the fluid out of the reservoir. The puncture mechanism is operable to puncture the wall structure and form a fluidic path between the reservoir and one or more channels. In certain embodiments, the channels are in fluid communication with a porous membrane configured to distribute the fluid to an area adjacent to and outside of the outer surface of the main housing. For example, the one or more channels may extend along the length of the porous membrane, such as in a helical or linear shape.

In certain embodiments, the puncture mechanism includes a channel in fluid communication with a needle tip oriented to puncture the wall structure of the reservoir. As shown in FIGS. 1A-1C, the first puncture mechanism **104** includes a channel in fluid communication with a needle tip oriented to puncture the first wall structure **102.** In certain embodiments, as shown in FIGS. 3A and 3B, the first puncture mechanism **304** includes an annular channel **305** in fluid communication with a needle tip **307** oriented to puncture the first wall structure **302.**

As shown in FIGS. 2A-2C, the device **200** includes first and second puncture mechanisms **204, 205** operable to puncture the first and second wall structures **202, 203,** respectively, and form a fluidic path between the first and second fluid reservoirs **214, 216,** and the first and second sets of channels **206, 207,** respectively, which are in fluid communication with the porous membrane **242.** First and second positive displacement mechanisms **222, 223** are operable, following puncture of the first and second wall structures **202, 203,** respectively, to drive the first and second fluids out of the first and second fluid reservoirs **214, 216,** through the first and second sets of channels **206, 207,** and into the porous membrane **242.** The porous membrane **242** is configured to distribute both the first and second fluids to the area adjacent to and outside of the outer surface of the main housing **212.** In one embodiment, as shown in FIGS. 3A and 3B, the one or more first channels **306** are distinct from the one or more second channels **308,** such that the fluidic paths for the first and second fluids may be isolated from one another. For example, separate sets of channels may decrease contamination.

In certain embodiments, the device includes a mechanism configured to allow a user to selectively cause the first puncture mechanism to puncture the first wall structure. As shown in FIGS. 1A-1C, the device **100** may include a push button **150** disposed on the outer surface of main housing **112,** such that it is accessible by a user. For example, the device may include a first tension loaded mechanism configured to advance the first reservoir towards the puncture mechanism. In certain embodiments, the device includes a single push button configured to cause all of the reservoirs in the device to be punctured, in series or parallel. For example, the device may be configured to puncture the reservoirs in series, according to a predetermined timing schedule.

In certain embodiments, the puncture mechanism includes a channel in fluid connection with the needle tip and configured to form a fluidic path between the channels disposed in or adjacent to the porous membrane. As shown in FIGS. 3A and 3B, the channel **305** of the first puncture mechanism **304** may be an annular channel. In certain embodiments, the channel of the puncture mechanism is sized and shaped to be adjacent the inner surface of the main housing. As shown in FIGS. 3A and 3B, the channel **305** of the first puncture mechanism **304** includes a radial aperture **310** in fluid communication with the one or more first channels **306,** which are in fluid communication with the porous membrane **342.** In certain embodiments, each puncture mechanism includes one or more apertures or fluid connections that provide the fluid communication with apertures or fluid connections in the channels of the porous membrane.

As shown in FIGS. 3A and 3B, the first and second annular channels **305, 312** may be positioned together in a stacked and/or offset arrangement within the interior region of the main housing.

For example, the needle tip of the puncture mechanism may be constructed of drug-compatible, biocompatible plastics or metals, such as stainless steel, and combinations thereof. The needle tip may be stiff or soft. For example, the needle tip may be stiff to assist in the puncturing process, or may be soft to assist in plugging the mechanism to prevent leakage once engaged with the reservoir. The channel of the puncture mechanism, as well as the channels in communication with the porous membrane, may be constructed of any drug-compatible, biocompatible tubing material, such as silicone, PTFE, polypropylene, and polyethylene, and/or metals, such as stainless steel.

Once the wall structure of the reservoir is breached by the puncture mechanism, i.e., by advancing the reservoir toward the puncture mechanism or advancing the puncture mechanism toward the reservoir, the positive displacement mechanism is actuated to drive the fluid out of the reservoir, through the fluidic path of the puncture mechanism and into the associated channels, which are in fluid communication with the porous membrane. For example, the lumenal surface of each of the one or more channels adjacent to or within the porous membrane may be porous, or include a plurality of outlets for providing a fluidic path between the channels and the porous membrane.

The porous membrane, or other common gate or valve structures used in place of or in addition to the porous membrane, may function to redirect or spread the fluid across a greater area of the region adjacent the device. For example, in a drug delivery device, the porous membrane sidewall may be configured to diffuse and distribute the drug formulations released from the reservoirs to the lumenal tissue. The porous membrane sidewall may be configured to distribute the drug formulations driven from the reservoirs to a tissue area adjacent the porous membrane sidewall when the device is deployed intralumenally in a human or animal subject.

The porous membrane may advantageously maximize the area which is exposed to the dispensed fluids by diffusing the fluids and distributing them across a large porous area on the outer surface of the device. The surface area of the porous membrane, and therefore the adjacent surface area to receive the fluid, may be adjusted depending upon the targeted fluid and delivery site. The porous membrane may also provide a reproducible wettable surface condition that reduces variability in the administered fluid and variability in the delivered fluid doses. Such features may be beneficial over conventional technologies having individual ports for each fluid to be dispensed. Such conventional devices may suffer from random delivery patterns and a lack of control over fluid and site exposure, resulting in highly variable dosing.

In one embodiment, the porous membrane operates as a fluidic valve. For example, the porous membrane may have a pore structure and chemistry such that a positive pressure is required to initiate flow of the fluid(s) through the porous membrane. This thresholding pressure may be tuned by controlling the average pore size of the membrane's pore structure, as well as the contact angle of the fluid on the surface of the membrane material. For example, the porous membrane sidewall may be a fluidic valve configured such that a critical threshold pressure from about 0,07 N/cm² to about 69 N/cm2 is required to distribute the fluids to the area adjacent the outer surface of the main housing.

The pore structure may be any microstructure representative of an open pore structure. This may be a single layer of pores that expend from one surface of the membrane through to the opposing surface of the membrane. Alternatively, the pore structure may be a randomly packed structure of interconnected pores or a highly ordered, closed packed pores structure. For example, the porous membrane may have an average pore size from about 0.2 µm to about 25 µm.

In certain embodiments, the porous membrane acts as an aseptic barrier. For example, the porous membrane may be configured to substantially prohibit infiltration into the device of bacteria having a size in excess of the effective average pore size of the porous membrane.

The porous membrane may be constructed of a polycarbonate, polypropylene, PFTE, or polyethylene membrane, or any combination of laminates thereof. The membrane may also be constructed of two or more dissimilar materials serving different functions outlined above. For example, a PTFE and polyethylene laminate structure may be used to achieve effective fluid solution spreading, antimicrobial delivery, and valving. Alternatively, a composite material may be constructed to achieve these desired functions for fluids having differing wetting characteristics. This may be achieved, for example, by using interwoven porous sheets constructed of a predetermined ratio of hydrophobic to hydrophilic materials.

In certain embodiments, upon generation of a positive pressure via the positive displacement mechanism, the fluids are driven from the reservoirs and through the porous membrane sidewall. Once the pressure is reduced, the wetting condition will become thermodynamically unfavorable and flow will stop.

In certain embodiments, the porous membrane provides a surface that is in primary contact with body tissue and therefore is composed of biocompatible materials. For example, the porous membrane may include a polypropylene membrane. Other suitable porous membrane materials include, but are not limited to, polyethersulfone, polycarbonate, polyethylene terephthalate, polyvinylidene fluoride, mixed cellulose ester, nylon 6,6, polytetrafluoroethylene, and combinations thereof.

In certain embodiments, the porous membrane substantially surrounds the main housing about the one or more reservoirs. For example, the porous membrane may be cylindrical. In certain embodiments, the porous membrane includes a first portion adjacent the release ends (i.e., the ends near the puncture mechanisms) of the reservoirs and a second portion adjacent the actuation ends of the reservoirs such that the drug formulations are distributed from both the first and second portions of the porous membrane. For example, the one or more channels may extend from a first end (i.e., at the release end) of the porous membrane to the opposed second end (i.e., at the actuation end) of the porous membrane.

For example, the devices described herein may include one or more of the release structure and/or multiple fluid delivery features described in U.S. Patent Applications No. 13/629,124 and 13/629,159.

### ACTUATION SYSTEM

In certain embodiments, the device includes one or more actuation systems which are configured to actuate the positive displacement mechanism(s) and in turn drive the fluid(s) from the reservoir(s). The one or more actuation systems may also be configured to actuate puncture of the wall structures of the reservoirs, i.e., by advancing the reservoir toward the puncture mechanism or advancing the puncture mechanism toward the reservoir. In one embodiment, a single actuation system may be configured to actuate multiple positive displacement mechanisms, so as to drive the first fluid from the first reservoir and drive the second fluid from the second reservoir, in series or parallel. In another embodiment, multiple actuation systems may be configured to actuate multiple positive displacement mechanisms so as to drive multiple fluids from multiple reservoirs.

The one or more actuation systems may be operably connected to the actuation ends of each of the reservoirs. Generally, each actuation system is configured to drive the fluid in the reservoir via a positive displacement process. The term "positive displacement," as used herein, refers to any process whereby the drug formulations are dispensed from the drug delivery device under force provided within each reservoir. Accordingly, the term does not refer to the passive, chemical diffusion of the drug formulations out of the reservoir, although passive diffusion may contribute to release of the drug formulations from the porous membrane.

As shown in FIG. 4B, the actuation system **738** may include a power source **740,** control circuitry **744,** and an actuation mechanism **746.** Embodiments having more than one actuation system may include multiple actuation mechanisms and a shared power source and control circuitry. Alternatively, embodiments having more than one actuation system may include multiple individual actuations systems, each having a power source, control circuitry, and actuation mechanism.

The power source may be any source of mechanical, electrical power or electromechanical power. The power source may include one or more batteries or fuel cells. The control circuitry may be configured to control the actuation system of the device, and thereby control the timing of release of the fluids. For example, the control circuitry may selectively transmit electrical and/or mechanical power to the actuation mechanism, positively displacing the fluid in the reservoirs. The control circuitry may be configured to control the timing of delivery of the fluids by applying the necessary electrical signals to the actuation mechanism. The control circuitry may be programmable or it may be pre-programmed to deliver the fluids in accordance with a prescribed (predetermined) release schedule.

The actuation mechanism may include fluid-volume displacement, mechanical displacement, osmotic swelling displacement, electrostatically-induced compression, piezoelectric actuation, thermally/magnetically induced phase transformation, or combinations thereof, to drive the fluids from the reservoirs via positive displacement. For example, the actuation mechanism may include one or more of the actuation mechanisms described in U.S. Patent Applications No. 13/629,124 and 13/629,159.

In certain embodiments, the one or more actuation systems are each configured to generate a displacement fluid in operable communication with an inflatable balloon structure within each reservoir. For example, the actuation system may include an electrolytic cell having a cathode and an anode which is in contact with water or an aqueous solution to generate a gas, such as oxygen, thus providing a source of positive displacement to drive the fluids out of the reservoirs.

In certain embodiments, the actuation system further includes a wireless receiver for receiving wireless control signals from a separate, detached transmitting device. For example, the fluid delivery device may be deployed into the lumen of a patient by the patient, physician, veterinarian, or the like, and thereafter, the patient, physician, veterinarian, or the like, may actuate the release of the fluids using the transmitting device to transmit control signals to the deployed device. Furthermore, in some embodiments, the receiver and transmitting device may both be transceivers capable of transmitting and receiving control signals and other communications from each other. For example, the receiver and transmitting device may be configured to allow for wireless programming and re-programming of the drug dosing regimen, including the dosage, timing of drug release, etc. Accordingly, in certain embodiments, the transceiver may transmit data relevant to the operation of the device, such as data regarding the fluids already administered, the release schedule, the amount of fluids remaining in the reservoirs, and the remaining battery charge, as well as data relevant to the environment of the deployment site, such as data detected or measured by an integral sensor. In some embodiments, the actuation system may also be wirelessly powered.

In certain embodiments, the device may is configured for wireless operation, e.g., following deployment in a human or animal subject. In such cases, the device includes appropriate telemetry components as known in the art. For example, actuation of the fluid dispensing may be done from a remote controller, e.g., external to a human or animal subject. Generally, the telemetry (i.e. the transmitting and receiving) is accomplished using a first coil to inductively couple electromagnetic energy to a matching/corresponding second coil. The means of doing this are well established, with various modulation schemes such as amplitude or frequency modulation used to transmit the data on a carrier frequency. The choice of the carrier frequency and modulation scheme will depend on the location of the device and the bandwidth required, among other factors. Other data telemetry systems known in the art also may be used. In another case, the device is configured to be remotely powered, or charged. For example, the device may include a transducer for receiving energy wirelessly transmitted to the device, circuitry for directing or converting the received power into a form that can be used or stored, and if stored, a storage device, such as a rechargeable battery or capacitor. In still another case, the device is both wirelessly powered and wirelessly controlled.

### DRUG FORMULATIONS

In certain embodiments, the fluid delivery device is an implantable drug delivery device for the controlled release of one or more drugs to an intralumenal tissue surface of a patient. In such embodiments, one or more drug formulations are contained within the device reservoirs for delivery to the mucosal tissue. In one embodiment, two or more drug formulations are disposed within two reservoirs for release to a subject.

Various drug formulations may be administered from the drug delivery device. The drug formulations within each reservoir may each include the same drug, may each include different drugs, or may be some combination of more than one similar drug and more than one different drug. For example, the first drug formulation may include a different drug than the second drug formulation. For example, the first and third drug formulations may both include the same drug, and second drug formulations may include a different drug than the first and third drug formulations. In certain embodiments, the device may be used to deliver a battery of drug formulations for a combination therapy, prophylaxis, or for another specific treatment, such as may be useful in animal husbandry.

In one embodiment, the device is used to deliver a fixed time artificial insemination treatment to a human or animal subject. In certain embodiments, the first drug formulation includes a gonadotropin-releasing hormone, the second drug formulation includes a prostaglandin, and the third drug formulation includes a gonadotropin-releasing hormone. In one embodiment, the device also includes a fourth drug formulation which includes a progestin. Variations of the drugs and sequences are envisioned.

In embodiments, the drug formulations include one or more proteins or peptides. For example, in some embodiments, the drug delivery device may be used to administer hormones or steroids, including, but not limited to, follicle stimulating hormone, parathyroid hormone, luteinizing hormone, gonadotropin-releasing hormone (GnRH), estradiol, progesterone, melatonin, serotonin, thyroxine, triiodothyronine, epinephrine, norepinephrine, dopamine, antimullerian hormone, adiponectin, adrenocorticotropic hormone, angiotensinogen, angiotensin, antidiuretic hormone, atrial-natriuretic peptide, calcitonin, cholecystokinin, corticotropin-releasing hormone, erythropoietin, gastrin, ghrelin, glucagon, growth hormone-releasing hormone, human chorionic gonadotropin, human placental lactogen, growth hormone, inhibin, insulin, insulin-like growth factor, leptin, melanocyte stimulating hormone, orexin, oxytocin, prolactin, relaxin, secretin, somatostatin, thrombopoietin, thyroid-stimulating hormone, thyrotropin-releasing hormone, cortisol, aldosterone, testosterone, dehydroepiandrosterone, androstenedione, dihydrotestosterone, estrone, estriol, calcitriol, calcidiol, prostaglandins, leukotrienes, prostacyclin, thromboxane, prolactin releasing hormone, lipotropin, brain natriuretic peptide, neuropeptide Y, histamine, endothelin, enkephalin, renin, and pancreatic polypeptide.

In some embodiments, the drug delivery device may be used to administer cytokine signaling molecules or immunomodulating agents that are used in cellular communication. These molecules commonly comprise proteins, peptides, or glycoproteins. Cytokine signaling molecules include, for example, the four -helix bundle family which include the IL-2 subfamily (e.g., erythropoietin (EPO) and thrombopoietin (THPO)), the interferon (IFN) subfamily and the IL-10 subfamily. Cytokine signaling molecules also include the IL-1, IL-18, and IL-17 families.

In some embodiments, the drug delivery device may be used to administer drug formulations for pain management, including, but not limited to, corticosteroids, opioids, antidepressants, anticonvulsants (antiseizure medications), non-steroidal anti-inflammatory drugs, COX2 inhibitors (e.g., rofecoxib and celecoxib), ticyclic antidepressants (e.g., amitriptyline), carbamazepine, gabapentin and pregabalin, codeine, oxycodone, hydrocodone, diamorphine, and pethidine.

In some embodiments, the drug delivery device may be used to administer cardiovascular drug formulations. Examples include B-type natriuretic peptide (BNP), atrial natriuretic peptide (ANP), atrial natriuretic factor (ANF), atrial natriuretic hormone (ANH), and atriopeptin. Cardiovascular drug formulations that may be administered by the device also include, for example, antiarrhythmic agents, such as Type I (sodium channel blockers), including quinidine, lidocaine, phenytoin, propafenone; Type II (beta blockers), including metoprolol; Type III (potassium channel blockers), including amiodarone, dofetilide, sotalol; Type IV (slow calcium channel blockers), including diltiazem, verapamil; Type V (cardiac glycosides), including adenosine and digoxin. Other cardiacvascular drug formulations that may be administered by the device include ACE inhibitors, such as, for example, captopril, enalapril, perindopril, ramipril; angiotensin II receptor antagonists, such as, for example, candesartan, eprosartan, irbesartan, losartan, telmisartan, valsartan; beta blocker; and calcium channel blocker.

The drug formulations may be formulated with one or more pharmaceutically acceptable excipients as needed to facilitate the drug's storage in and release from the device. In one embodiment, the drug may be in a liquid solution or suspension. The drug may be in the form of microparticles or nanoparticles. The solvent or carrier may be aqueous or organic. For example, the devices and methods described herein may further include a reconstitution mechanism as described in U.S. Patent Application No. 13/629,184.

In some embodiments, the drug formulations may include components that are degradable by the enzymes present in the fluid secreted by the mucosal tissue. For example, certain amino acids present in drug formulations may be degraded by the enzymes present in fluid secreted by the mucosal tissue. Accordingly, the devices and methods described herein may further include one or more of the permeation enhancement mechanisms described in U.S. Patent Application Publications No. 2011/0087195, No. 2011/0087192, and No. 2011/0087155.

### METHODS

Methods are provided for controlled delivery of fluid(s) to a target area. The devices may include any combination of the device feature described herein. In certain embodiments, as shown in FIGS. 1A-1C the method includes: (i) positioning a fluid delivery device **100** adjacent to the target area, (ii) puncturing the first wall structure **102** with a first puncture mechanism **104** to provide a fluidic path between the first fluid reservoir **114** and one or more first channels **106** in fluid communication with the porous membrane **142,** and (iii) driving the first fluid out of the first fluid reservoir **114** and into the one or more first channels **106,** such that the first fluid is delivered to the target area from the device **100** via the porous membrane **142.**

In certain embodiments, the methods include deploying a drug delivery device into the mucosal lumen of a human or animal patient. For example, the subject may be a mammalian animal (e.g., cow, sheep, horse, pig, or dog). The methods include various medical and veterinary therapies, as well as animal husbandry applications. The lumen may be, for example, a vagina, cervix, uterus, bladder, or rectum. The device may be adapted to contact essentially any mucosal tissue surface. The device may be placed in the lumen by inserting the device through an exterior orifice of the patient into the lumen. In some embodiments, the device may be in a form that may be orally administered for delivery of a drug via the mucosal tissue of the gastrointestinal tract.

In certain embodiments, the first wall structure is punctured prior to positioning the fluid delivery device adjacent to the target area. For example, as shown in FIGS. 1A-1C, a user may press the button **150,** to actuate puncturing of the wall structure **102,** prior to positioning the device within a bodily lumen **160.** In other embodiments, the puncturing of one or more wall structures may be actuated wirelessly after the device has been positioned.

In certain embodiments, driving the fluid out of the fluid reservoir includes generating a gas to inflate the balloon within the fluid reservoir and displace the fluid. In other embodiments, the device further includes a balloon containing the first fluid disposed in the first fluid reservoir, and driving the first fluid out of the first fluid reservoir includes generating a gas to deflate the balloon and displace the first fluid. For example, in devices having multiple reservoirs, the fluid in the first reservoir may be completely or partially released from the first reservoir before the release of the fluid from the second reservoir.

As illustrated in FIG. 1, the fluid delivery device **100** may be placed in a lumen 160. The device may be held in place by frictional engagement between the mucosal tissue and the main housing. As shown in FIGS. 4A-4B, arms **750** may be provided to facilitate retention of the device within the mucosal lumen. The fluids may then be driven out of the reservoirs and into the porous membrane **742** from which the drug formulations are then released to the surrounding area. The actuation of the actuation system may be controlled by the control circuitry **744.** The device may thereafter be removed from the lumen. For example, the devices described herein may include one or more of the retention features described in U.S. Patent Application No. 13/742,203.

The fluid delivery devices may include any of the device features described herein. For example, the device may include a microcontroller configured to control the actuation system, and thereby control the timing of the release of the fluids.

### APPLICATIONS/USES

The fluid delivery devices and methods may be used for any applications in which controlled fluid delivery to a target area is needed. For example, the fluid delivery devices and methods may be used for various medical and therapeutic applications in human and animal subjects, as well as in animal husbandry. In certain embodiments, the fluids are drug formulations.

In one embodiment, an implantable drug delivery device for the controlled release of two or more drugs to an intralumenal tissue surface is provided. The device includes a main housing configured for intralumenal deployment in a patient and having an outer surface which includes a porous membrane. First and second fluid reservoirs are contained within the main housing, and contain a first drug and a second drug, respectively. The first and second drugs may be the same drug formulation or different drug formulations. A first puncture mechanism is operable to puncture the first fluid reservoir and form a fluidic path between the first fluid reservoir and one or more first channels, which are in fluid communication with the porous membrane. A second puncture mechanism is operable to puncture the second fluid reservoir and form a fluidic path between the second fluid reservoir and one or more second channels, which are also in fluid communication with the porous membrane. At least one positive displacement mechanism is operable to (i) drive the first fluid out of the first fluid reservoir, through the one or more first channels, and into the porous membrane following puncture of the first fluid reservoir, and (ii) drive the second fluid out of the second fluid reservoir, through the one or more second channels, and into the porous membrane following puncture of the second fluid reservoir. The porous membrane is operable to distribute the first and second drugs to an intralumenal tissue surface adjacent to the outer surface of the main housing.

In some embodiments, the drug delivery device may be used to treat infertility or provide a fixed time artificial insemination (FTAI) treatment in a female subject. For example, the drug delivery device may be placed in the vagina (or uterus, or other part of the birth canal) of a female subject. The drug delivery device may then deliver follicle stimulating hormone to induce ovulation in the female subject. In some embodiments, the drug delivery device may be configured to deliver a plurality of hormones, including follicle stimulating hormone, luteinizing hormone, gonadotropin-releasing hormone separately, or in combination, in appropriate sequences, at appropriate times, and in pharmacologically appropriate amounts. The device may also dispense estradiol to regulate natural hormone production in the female subject. The appropriate dosing schedule and amounts may be determined by one in the field of reproductive pharmacology.

Compared to traditional FTAI treatments, the methods described herein may require only device implantation and removal at the time of artificial insemination, and result in a reduction in time spent driving, herding and chuting cattle. The methods also result in improved ovulation quality and quantity due to the reduction in handling, stress, and systemic cortisol levels of the subjects. The methods also reduce the number of medical supplies needed, as a single device delivery the series of FTAI drugs.

In another embodiment, the drug delivery device may be used to treat insulin dependent diabetes (Type I diabetes) in a subject. The drug delivery device may be placed within a lumen of the subject. The drug delivery device may then deliver insulin (Humulin R, Novolin R), insulin isophane (Humulin N, Novolin N), insulin lispro (Humalog), insulin aspart (NovoLog), insulin glargine (Lantus) or insulin detemir (Levemir) to the patient at a selected time or times.

In another embodiment, the drug delivery device may be used to treat diabetes mellitus (Type II diabetes) in a subject. The drug delivery device may be placed within a lumen of the subject. The drug delivery device may then deliver exenatide to the patient at a selected time or times.

In another embodiment, the drug delivery device may be used to treat breast or ovarian cancer in a subject. The drug delivery device may be placed within a lumen of the subject, such as the vagina for a female subject. The drug delivery device may then deliver abraxane (or other drug effective in the treatment or management of cancer) to the patient at a selected time or times.

In another embodiment, the drug delivery device may be used to treat HIV/AIDS in a subject. The drug delivery device may be placed within a lumen of the subject. The drug delivery device may then deliver Abacavir (ABC) or Cidofovir (or other drug effective in the treatment or management of HIV/AIDS) to the patient at a selected time or times. The device also may be used to treat other sexually transmitted diseases.

In another embodiment, the drug delivery device may be used to treat genital herpes in a subject. The drug delivery device may be placed within a lumen of the subject, such as within the vagina of a female subject. The drug delivery device may then deliver acyclovir, famciclovir, or valacyclovir (or other drug effective in the treatment or management of genital herpes) to the patient at a selected time or times.

In another embodiment, the drug delivery device may be used to treat diabetes insipidus in a subject. The drug delivery device may be placed within a lumen of the subject. The drug delivery device may then deliver desmopressin (or other drug effective in the treatment or management of diabetes insipidus) to the patient at a selected time or times.

In another embodiment, the drug delivery device may be used to treat osteoporosis in a subject. The drug delivery device may be placed within a lumen of the subject, such as within the vagina of a female subject. The drug delivery device may then deliver ibandronate, calcitonin, or parathyroid hormone (or other drug effective in the treatment or management of osteoporosis) to the patient at a selected time or times.

Overall, the devices and methods described herein provide a means to deliver multiple fluids through a common distribution means (e.g., gate, valve, porous membrane). The devices take advantage of the common porous membrane, while providing the ability to engage the reservoir-membrane fluidic connection just before use. Thus, this engaging mechanism allows the reservoirs to remain unbreached during shipping and on the shelf, thus maintaining the chemical and biological integrity of the fluids contained therein, such as drugs. Moreover, the device structure allows each fluid to be delivered via individual and isolated channels, thereby enabling independent access to the common membrane for each fluid dispensed and reducing the potential for contamination.

It will be appreciated that various of the above-disclosed and other features and functions, or alternatives thereof, may be desirably combined into many other different devices, methods, or applications. Various presently unforeseen or unanticipated alternatives, modifications, variations, or improvements therein may be subsequently made by those skilled in the art which are also intended to be encompassed by the following claims.

## Claims

1. A fluid delivery device (100), comprising:
a main housing (112) having an interior region and having an outer surface which comprises a porous membrane (142);
a first fluid reservoir (114) disposed in the interior region of the main housing (112), the first fluid reservoir (114) being formed at least in part by a first wall structure (102) and containing a first fluid;
a first puncture mechanism (104) operable to puncture the first wall structure (102) and form a fluidic path between the first fluid reservoir (114) and one or more first channels (106), which are in fluid communication with the porous membrane (142); and
a first positive displacement mechanism (122) operable, following puncture of the first wall structure (102), to drive the first fluid out of the first fluid reservoir (114), through the one or more first channels (106), and into the porous membrane (142), wherein the porous membrane (142) is configured to distribute the first fluid to an area adjacent to and outside of the outer surface of the main housing (112).

2. The device of claim 1, wherein:
the first fluid is contained within a balloon disposed in the first fluid reservoir (114), and the first positive displacement mechanism (122) comprises a gas generating mechanism configured to generate a gas to deflate the balloon and displace the first fluid.

3. The device of claim 1, wherein the first puncture mechanism (104) comprises an annular channel in fluid communication with a needle tip oriented to puncture the first wall structure (102).

4. The device of claim 1, further comprising:
a second fluid reservoir disposed in the interior region of the main housing (112), the second fluid reservoir being formed at least in part by a second wall structure and containing a second fluid;
a second puncture mechanism operable to puncture the second wall structure and
form a fluidic path between the second fluid reservoir and one or more second channels, which are in fluid communication with the porous membrane (142); and
a second positive displacement mechanism operable, following puncture of the second wall structure, to drive the second fluid out of the second fluid reservoir, through the one or more second channels, and into the porous membrane (142), wherein the porous membrane (142) is configured to distribute the second fluid to an area adjacent to and outside of the outer surface of the main housing (112).

5. The device of claim 4, wherein the one or more second channels are distinct from the one or more first channels (106).

6. The device of claim 4, wherein:
the first fluid reservoir (114) and the second fluid reservoir each comprise an elongated annular tube with a closed end wall, and the first wall structure (102) and the second wall structure respectively comprise the closed end walls of the first fluid reservoir (114) and the second fluid reservoir.

7. The device of claim 6, wherein:
the first puncture mechanism (104) comprises a first annular channel in fluid communication with a first needle tip configured to puncture the first wall structure (102), the second puncture mechanism comprises a second annular channel in fluid communication with a second needle tip configured to puncture the second wall structure, and the first and second annular channels are positioned together in a stacked and/or offset arrangement within the interior region.

8. The device of claim 1, wherein the porous membrane (142) comprises a fluidic valve configured such that a critical threshold pressure from about 0.07 N/cm² to about 69 N/cm² is required to distribute the first fluid to the area adjacent to and outside of the outer surface of the main housing (112).

9. The device of claim 1, wherein the porous membrane (142) has an average pore size from about 0.2 µm to about 25 µm.

10. The device of claim 4,
wherein the first fluid comprises a first drug;
the second fluid comprises a second drug; and
the porous membrane (142) is operable to distribute the first and second drugs to an intralumenal tissue surface adjacent to the outer surface of the main housing (112).

## Patentansprüche

1. Fluidabgabevorrichtung (100), die Folgendes umfasst:
ein Hauptgehäuse (112) mit einem Innenbereich und einer Außenfläche, die eine poröse Membran (142) umfasst,
einen ersten Fluidbehälter (114), der in dem Innenbereich des Hauptgehäuses (112) angeordnet ist, wobei der erste Fluidbehälter (114) zumindest teilweise von einer ersten Wandkonstruktion (102) gebildet wird und ein erstes Fluid enthält,
einen ersten Durchstechmechanismus (104), der dazu dient, die erste Wandkonstruktion (102) zu durchstechen und zwischen dem ersten Fluidbehälter (114) und einem oder mehreren ersten Kanälen (106), die mit der porösen Membran (142) in Fluidverbindung stehen, einen fluidischen Pfad zu bilden, und
einen ersten Verdrängungsmechanismus (122), der dazu dient, nach dem Durchstechen der ersten Wandkonstruktion (102) das erste Fluid über den einen oder die mehreren ersten Kanäle (106) aus dem ersten Fluidbehälter (114) in die poröse Membran (142) zu drücken, wobei die poröse Membran (142) so konfiguriert ist, dass sie das erste Fluid in einen Bereich neben und außerhalb der Außenfläche des Hauptgehäuses (112) verteilt.

2. Vorrichtung nach Anspruch 1, wobei:
das erste Fluid in einem Ballon enthalten ist, der in dem ersten Fluidbehälter (114) angeordnet ist, und der erste Verdrängungsmechanismus (122) einen Gaserzeugungsmechanismus umfasst, der so konfiguriert ist, dass er ein Gas zum Entleeren des Ballons und Verdrängen des ersten Fluids erzeugt.

3. Vorrichtung nach Anspruch 1, wobei der erste Durchstechungsmechanismus (104) einen ringförmigen Kanal umfasst, der mit einer Nadelspitze in Fluidverbindung steht, die zum Durchstechen der ersten Wandkonstruktion (102) ausgerichtet ist.

4. Vorrichtung nach Anspruch 1, die ferner Folgendes umfasst:
einen zweiten Fluidbehälter, der im Innenbereich des Hauptgehäuses (112) angeordnet ist, wobei der zweite Fluidbehälter zumindest teilweise von einer zweiten Wandkonstruktion gebildet wird und ein zweites Fluid enthält,
einen zweiten Durchstechmechanismus, der dazu dient, die zweite Wandkonstruktion zu durchstechen und zwischen dem zweiten Fluidbehälter und einem oder mehreren zweiten Kanälen, die mit der porösen Membran (142) in Fluidverbindung stehen, einen fluidischen Pfad zu bilden, und
einen zweiten Verdrängungsmechanismus, der dazu dient, nach dem Durchstechen der zweiten Wandkonstruktion das zweite Fluid über den einen oder die mehreren zweiten Kanäle aus dem zweiten Fluidbehälter in die poröse Membran (142) zu drücken, wobei die poröse Membran (142) so konfiguriert ist, dass sie das zweite Fluid in einen Bereich neben und außerhalb der Außenfläche des Hauptgehäuses (112) verteilt.

5. Vorrichtung nach Anspruch 4, wobei sich der eine oder die mehreren zweiten Kanäle von dem einen oder den mehreren ersten Kanälen (106) unterscheiden.

6. Vorrichtung nach Anspruch 4, wobei:
der erste Fluidbehälter (114) und der zweite Fluidbehälter jeweils ein längliches, ringförmiges Röhrchen mit einer geschlossenen Stirnwand umfassen und die erste Wandkonstruktion (102) beziehungsweise die zweite Wandkonstruktion die geschlossene Stirnwand des ersten Fluidbehälters (114) beziehungsweise des zweiten Fluidbehälters umfasst.

7. Vorrichtung nach Anspruch 6, wobei:
der erste Durchstechungsmechanismus (104) einen ersten ringförmigen Kanal umfasst, der mit einer ersten Nadelspitze in Fluidverbindung steht, die so konfiguriert ist, dass sie die erste Wandkonstruktion (102) durchsticht, der zweite Durchstechungsmechanismus einen zweiten ringförmigen Kanal umfasst, der mit einer zweiten Nadelspitze in Fluidverbindung steht, die so konfiguriert ist, dass sie die zweite Wandkonstruktion durchsticht, und der erste und der zweite ringförmige Kanal zusammen in einer gestapelten und/oder versetzten Anordnung im Innenbereich positioniert sind.

8. Vorrichtung nach Anspruch 1, wobei die poröse Membran (142) ein fluidisches Ventil umfasst, das so konfiguriert ist, dass zum Verteilen des ersten Fluids in den Bereich neben und außerhalb der Außenfläche des Hauptgehäuses (112) ein kritischer Schwellendruck von etwa 0,07 N/cm² bis etwa 69 N/cm² erforderlich ist.

9. Vorrichtung nach Anspruch 1, wobei die poröse Membran (142) eine durchschnittliche Porengröße von etwa 0,2 µm bis etwa 25 µm aufweist.

10. Vorrichtung nach Anspruch 4,
wobei das erste Fluid ein erstes Medikament umfasst,
das zweite Fluid ein zweites Medikament umfasst und
die poröse Membran (142) dazu dient, das erste und das zweite Medikament auf einer an die Außenfläche des Hauptgehäuses (112) angrenzenden intraluminalen Gewebeoberfläche zu verteilen.

## Revendications

1. Dispositif de délivrance de fluide (100), comprenant :
un logement principal (112) ayant une région intérieure et ayant une surface externe qui comprend une membrane poreuse (142) ;
un premier réservoir de fluide (114) disposé dans la région intérieure du logement principal (112), le premier réservoir de fluide (114) étant formé au moins en partie par une première structure de paroi (102) et contenant un premier fluide ;
un premier mécanisme de perforation (104) pouvant fonctionner pour perforer la première structure de paroi (102) et former une trajectoire fluidique entre le premier réservoir de fluide (114) et un ou plusieurs premiers canaux (106), qui sont en communication fluidique avec la membrane poreuse (142) ; et
un premier mécanisme de déplacement positif (122) pouvant fonctionner, suite à la perforation de la première structure de paroi (102), pour entraîner le premier fluide hors du premier réservoir de fluide (114), à travers les un ou plusieurs premiers canaux (106), et jusque dans la membrane poreuse (142), dans lequel la membrane poreuse (142) est configurée pour distribuer le premier fluide vers une zone adjacente et extérieure à la surface externe du logement principal (112).

2. Dispositif selon la revendication 1, dans lequel :
le premier fluide est contenu au sein d'un ballonnet disposé dans le premier réservoir de fluide (114), et le premier mécanisme de déplacement positif (122) comprend un mécanisme de génération de gaz configuré pour générer un gaz pour dégonfler le ballonnet et déplacer le premier fluide.

3. Dispositif selon la revendication 1, dans lequel le premier mécanisme de perforation (104) comprend un canal annulaire en communication fluidique avec une pointe d'aiguille orientée pour perforer la première structure de paroi (102).

4. Dispositif selon la revendication 1, comprenant en outre :
un deuxième réservoir de fluide disposé dans la région intérieure du logement principal (112), le deuxième réservoir de fluide étant formé au moins en partie par une deuxième structure de paroi et contenant un deuxième fluide ;
un deuxième mécanisme de perforation pouvant fonctionner pour perforer la deuxième structure de paroi et former une trajectoire fluidique entre le deuxième réservoir de fluide et un ou plusieurs deuxièmes canaux, qui sont en communication fluidique avec la membrane poreuse (142) ; et
un deuxième mécanisme de déplacement positif pouvant fonctionner, suite à la perforation de la deuxième structure de paroi, pour entraîner le deuxième fluide hors du deuxième réservoir de fluide, à travers les un ou plusieurs deuxièmes canaux, et jusque dans la membrane poreuse (142), dans lequel la membrane poreuse (142) est configurée pour distribuer le deuxième fluide vers une zone adjacente et extérieure à la surface externe du logement principal (112).

5. Dispositif selon la revendication 4, dans lequel les un ou plusieurs deuxièmes canaux sont distincts des un ou plusieurs premiers canaux (106).

6. Dispositif selon la revendication 4, dans lequel :
le premier réservoir de fluide (114) et le deuxième réservoir de fluide comprennent chacun un tube annulaire allongé avec une paroi d'extrémité fermée, et la première structure de paroi (102) et la deuxième structure de paroi comprennent respectivement les parois d'extrémité fermée du premier réservoir de fluide (114) et du deuxième réservoir de fluide.

7. Dispositif selon la revendication 6, dans lequel :
le premier mécanisme de perforation (104) comprend un premier canal annulaire en communication fluidique avec une première pointe d'aiguille configurée pour perforer la première structure de paroi (102), le deuxième mécanisme de perforation comprend un deuxième canal annulaire en communication fluidique avec une deuxième pointe d'aiguille configurée pour perforer la deuxième structure de paroi, et les premier et deuxième canaux annulaires sont positionnés ensemble dans un agencement empilé et/ou décalé au sein de la région intérieure.

8. Dispositif selon la revendication 1, dans lequel la membrane poreuse (142) comprend une soupape fluidique configurée de sorte qu'une pression de seuil critique d'environ 0,07 N/cm² à environ 69 N/cm² est nécessaire pour distribuer le premier fluide vers la zone adjacente et extérieure à la surface externe du logement principal (112).

9. Dispositif selon la revendication 1, dans lequel la membrane poreuse (142) a une taille moyenne de pores d'environ 0,2 µm à environ 25 µm.

10. Dispositif selon la revendication 4,
dans lequel le premier fluide comprend un premier médicament ;
le deuxième fluide comprend un deuxième médicament ; et
la membrane poreuse (142) peut fonctionner pour distribuer les premier et deuxième médicaments à une surface de tissu intraluminal adjacente à la surface externe du logement principal (112).
